# EUROPEAN PATENT APPLICATION

(11) **EP 4 844 381 A1**
(43) Date of publication of application: **25.11.2026**
(21) Application number: 25218932.9
(22) Date of filing: 27.11.2025
(51) Int. Cl.: G01N 21/77, G01N 21/81, G01N 33/38, G01N 17/00

(54) **OPTICAL FIBRE SENSOR FOR STRUCTURAL HEALTH MONITORING OF REINFORCED CONCRETE STRUCTURES, METHODS AND USES THEREOF**

(30) Priority: 23.05.2025 PT 2025120285
(71) Applicant: INESC TEC - Instituto de Engenharia de Sistemas e Computadores, Tecnologia e Ciência, 4200-465 Porto (PT)
(72) Inventor: MADEIRA DA SILVA, PEDRO MIGUEL, 4169-007 PORTO (PT); COSTA COELHO, LUÍS CARLOS, 4169-007 PORTO (PT); MARQUES MARTINS DE ALMEIDA, JOSÉ MANUEL, 4169-007 PORTO (PT); SAMPAIO MENDES, JOÃO PEDRO, 4169-007 PORTO (PT)
(74) Representative: Patentree

(57) **Abstract**

The present disclosure relates to an optical fibre sensor for monitoring the integrity of a reinforced concrete structure, comprising: a bundle of multimode optical fibres to be embedded in the concrete structure, said bundle comprises: a first bare fibre configured to detect changes in refractive index related with concrete curing and water ingress; a second fibre having a tip with coated in or embedded with a calibrated non-carbonated cement layer, said second fibre is configured to detect carbonation by monitoring variations in reflectance; a third fibre comprising a tip coated with a suitable porous material, preferably a carbonated cement layer and configured to detect relative humidity changes within the concrete; a fourth fibre having a tip coated with an iron-based thin film said tip is further coated with or embedded in a suitable porous material, preferably a cement paste, said fourth fibre is configured to detect corrosion through multi-wavelength reflectance measurement; at least one light source optically coupled to each fibre of the fibre bundle; at least one photodetector optically coupled to each fibre of the fibre bundle and configured to receive reflected signals; a processing unit configured to analyse the reflected light intensities from the fibres and to correlate the detected signals with each structural integrity indicator.

## Description

### TECHNICAL FIELD

The present disclosure relates to the field of structural health monitoring, and more specifically to low-cost optical fibre sensors for the in-situ monitoring of reinforced concrete structures (RCS). Namely to an optical fibre sensor for detecting/monitoring curing behaviour, water intrusion, relative humidity, carbonation, and corrosion within RCS using functionalized multimode optical fibres.

### BACKGROUND

Structural health monitoring (SHM) of reinforced concrete structures (RCS) remains reliant on visual and periodic inspections, which are insufficient for monitoring the ever-expanding stock of aging infrastructure.

Reinforced Concrete Structures (RCS) have reshaped our society, enabling the expansion of metropolises, improving the quality of housing, shortening travel distances, and supporting essential services. The demand for reinforced concrete is expected to continue. Emerging economies are focused on expanding their infrastructures, while developed nations are tasked with repairing their aging infrastructure [1].

The longevity of reinforced concrete overshadows the complex and imminent problem of its decay. Mechanisms of decay, such as cement paste carbonation, chloride ion ingress, freeze-thaw cycles, alkali-silica reaction, water ingress, and rebar corrosion, influence and reinforce one another [2]. Mitigating the economic, social, and environmental impacts of reinforced concrete decay is only possible through its monitoring.

Reinforced concrete decay is highly heterogeneous, requiring high-granularity sensors for proper structural assessment and, consequently, low-cost sensors. Embedding a sensor during the curing of reinforced concrete requires it to withstand high temperatures, high relative humidity (RH), elevated pH, and strong vibrations caused by compacting the concrete mixture.

Optical fibre sensors have been used to monitor RCS, although bulky and costly interrogation systems often limit their adoption in the industry. Operating in the visible range with multimode fibres (MMF) significantly reduces costs due to less expensive components and simpler light coupling. This approach has been employed for low-cost monitoring of cement paste curing, RH, water intrusion, and carbonation [3-5]. It has also been used to monitor rebar corrosion in RCS [6]. Monitoring these parameters is crucial because curing ensures the concrete's desired structural properties and longevity. Water influences curing and several decay mechanisms [2,3]. Cement paste carbonation reduces the pH of the cement paste, exposing rebars to corrosion, which in turn causes spalling and further exposure to the elements. In this work, these sensors are adapted to monitor RCS and bundled together for comprehensive monitoring.

Micro-Electro-Mechanical Systems, electrical, and electrochemical sensors are not capable of enduring the adverse environment of concrete due to the high temperatures and water present during the cure, the lasting high RH that exists inside concrete, the possibility of electromagnetic interference, the possibility of water intrusion, the high pH, physical stress and pressure and their lack of long-term stability.

These facts are disclosed in order to illustrate the technical problem addressed by the present disclosure.

### GENERAL DESCRIPTION

The present disclosure relates to an optical fibre sensor system for monitoring structural health in reinforced concrete structures. The system includes a bundle of multimode optical fibres, each functionalized to detect different degradation parameters such as curing, water intrusion, relative humidity, carbonation, and rebar corrosion. The fibres are interrogated using low-cost LEDs in the visible spectrum and integrated with photodetectors and 3D-printed holders for field durability. A multi-wavelength detection approach enables discrimination between corrosion and mechanical noise. The disclosure enables scalable, real-time, in-situ monitoring of concrete infrastructure using an embedded and cost-efficient optical sensing platform.

The present disclosure provides a compact, low-cost optical fibre sensor system capable of simultaneously monitoring multiple critical degradation parameters in reinforced concrete structures using visible-light multimode fibres. The integration of functionalized fibre tips with wavelength-specific interrogation and a multi-sensor assembly in a rugged, embedded format represents a non-obvious advancement over prior art, enabling reliable in-situ monitoring under harsh curing and operational conditions.

Reinforced concrete structures form the backbone of civil infrastructure due to their durability, longevity, affordability, and availability. However, aging concrete poses challenges, with decay often beginning internally and becoming visible only at advanced stages, leading to costly repairs, restricted functionality, and safety risks. To address these challenges, the present disclosure relates to an optical fibre sensor that comprises multimode fibres to measure changes in reflection that contain information on concrete structural health.

The solution of the present disclosure relates to a multimode optical fibre-based sensing of curing, humidity, water ingress, carbonation, and corrosion within concrete using reflectance-based interrogation. With the present disclosure, infrastructure resilience is enhanced, and repairs are optimized.

The optical fibre sensor, based on reflection measurements, is used for structural health monitoring of reinforced concrete by monitoring water ingress/egress, relative humidity (RH) changes, carbonation, cure, and corrosion of concrete.

The range of parameters being monitored in a single sensor is unmatched in the current market.

Working in the visible range of light, employing inexpensive LEDs and photodiodes allows for a lower price when compared with other OFS.

For monitoring concrete cure, water ingress/egress, cement paste carbonation, and RH, the sensing elements are optical fibres and cement paste or concrete. The inert properties of the optical fibre and the resilience of concrete and cement paste contribute to this sensor's longevity and stability, allowing the monitoring of concrete's life cycle.

The fibre tip coated in cement paste and placed within a small cavity in the structure to avoid direct contact with the surrounding concrete, has a higher affinity towards higher RH values due to cement paste's intrinsic porosity. Moreover, it can measure varying condensation levels and even submerge in water, which is one significant difficulty for current RH sensors.

The cement paste used in monitoring water adsorption for different RH values can be replaced by other porous materials, including, but not limited to, ceramics, whose intrinsic porosity allows for varying ranges of monitoring RH.

By monitoring water ingress and egress and the location of the fibre in the structure, it is possible to estimate the rate of water uptake as water passes through the fibre core.

In an embodiment, the multimode fibre tip is coated with iron (Fe), exploiting different LEDs to monitor changes in the absorption spectrum of the iron, which allows the monitoring of the onset of corrosion. Surprisingly, this monitoring of the onset corrosion results by embedding the thin film coated optical fibre in cement paste, which holds the corrosion by Fe products in place. These colorimetric measurements also increase the resilience of the measurements against mechanical variability.

By employing, at least, three fibres with different degrees of interaction with the structure, the present solution can resolve the varying phenomena that influence changes in reflected light intensity in a single embedded fibre (water intrusion, RH, carbonation). It allows for the calibration of the sensor before it is embedded and for discerning each of the measurements.

It is possible to monitor water throughout the entire service life of the structure, beginning with the cure. Reflection measurements for the monitoring of water in the process of concrete cure are not susceptible to changes in ambient temperature and can be performed in samples of any size, shape, or form. After the cure, the fibre can be left in place to monitor water ingress and egress throughout the structure's lifecycle due to the fibre's longevity, even in an environment such as concrete.

The RH sensors employ a fibre coated in a small piece of cement paste, have an affinity towards higher RH values, and can measure beyond 100% RH, such as condensation and water intrusion, which usually leads to the impairment of current RH sensors.

In an embodiment, the fibre has a diameter of 200 to 1000 µm, preferably from 300 to 700 µm, more preferably 600 µm.

By using a small sample of cement paste cured in the fibre tip, it is possible to monitor cement paste carbonation directly through the changing colour of the paste due to the formation of calcium carbonate. The longevity of the paste mirrors that of the structure, which is indispensable in monitoring concrete carbonation, a process that takes years to occur. Changes in the amount of water in the cement paste are a source of variability that can be measured through the water ingress and RH tips and ultimately removed.

In an embodiment, by placing on the fibre tip an iron-base thin film encased in cement paste, corrosion occurs in the vicinity of the fibre tip, increasing the sensor's sensibility and resilience. Colorimetric changes that occur in the early stages of corrosion can be measured, allowing for earlier warning. False positives from mechanical changes can be averted by measuring in the spectrum.

In an embodiment, by placing a fibre tip in concrete, light reflected at the tip will be susceptible to changes in the pores' refractive index (RI) as they fill and empty out with water. A bare fibre embedded in the concrete will monitor all processes that influence the amount of water near the fibre tip, namely the cure of concrete, changes in RH, and ingress/egress of water. A bare fibre is susceptible to changes in the concrete structure near the core, namely the formation of cracks and the discoloration of the concrete due to carbonation. By increasing the number of fibres, preferably to three or four, it is possible to interrogate the different measurands by changing the degree of interaction each has with concrete.

In an embodiment, one fibre is coated with carbonated cement paste and kept separate from the structure to measure only relative humidity (RH) changes. Another fibre is coated with or embedded in non-carbonated cement paste before being embedded in the concrete, enabling measurement of cement paste carbonation. A third fibre is placed directly onto the concrete to measure both the same parameters and changes in liquid water within the cement paste pores, resulting from water ingress or egress, as well as the concrete curing process. Finally, a fourth fibre, coated with a thin iron-based film and embedded in or coated with a protective porous matrix, monitors the potential for corrosion in the structure by detecting the colorimetric changes that occur as iron oxidizes.

In an embodiment, the porous matrix is the cement paste with a porosity between 25-50%, preferably 30-45%; wherein the porous size ranges from 1-200 nm; preferably 50-100 nm. The porosity and porous size may be measured by many methods. In the present disclosure the measurement was carried out was measure by Gravimetric Method.

The present disclosure provides an optical fibre sensor for embedded monitoring of reinforced concrete structures. The sensor comprises a bundle of multimode optical fibres, each configured to detect a specific degradation or curing parameter through reflectance measurements at a functionalised fibre tip. Namely, a first bare optical fibre measures changes in refractive index caused by concrete curing or water ingress; a second optical fibre, whose tip is coated with or embedded in a non-carbonated cement layer, is configured to detect carbonation by monitoring reflectance variations; a third optical fibre includes a tip coated with a porous carbonated cement layer that responds to relative humidity variations; a fourth optical fibre, coated with an iron-based thin film and further coated or embedded in a cement layer, detects corrosion through multi-wavelength reflectance. Surprisingly, it was found that combining bare, cement-coated, porous-coated, and iron-film-coated multimode optical fibres within a single embedded reflectance sensor enables simultaneous and reliable detection of curing, humidity, carbonation, and corrosion in reinforced concrete allowing a comprehensive and continuous structural health assessment.

The solution further includes light sources optically coupled to each fibre, photodetectors for receiving reflected light, and a processing unit that correlates reflectance changes with structural indicators such as curing state, humidity, carbonation level, and corrosion progression.

Preferred embodiments include specific film thicknesses, cement compositions, coating thicknesses, interrogation strategies (single-wavelength for the first three fibres and multi-wavelength for the fourth), resin-coated fibre bundles, and alignment supports such as a plate, rod, and polymeric mesh for proper installation in concrete.

The present disclosure also provides a method of manufacturing the sensor, including calibration of each fibre by exposing it to controlled reference conditions (air, water, humidity) and monitoring reflected intensity. A method of using the sensor for structural health monitoring is also described, comprising embedding the fibre tips into concrete structure to be monitored, interrogating the fibres, and analysing reflected signals to detect curing, water ingress, carbonation, humidity, and corrosion.

In an embodiment, the sensor is configured to operate the fourth fibre using at least one light source emitting at two or more wavelengths.

In an embodiment, calcium carbonate accumulates in the pores of the cement of the tip of the second fibre.

In an embodiment, water accumulates in the pores of the carbonate cement of the tip of the third fibre.

In an embodiment, iron oxide accumulates in the pores of the cement of the tip of the fourth fibre.

In an embodiment, the tip of the second fibre is embedded in or coated with the calibrated non-carbonated concrete layer. The non-carbonated cement layer can be a cement type I- ordinary Portland cement or cement type II - ordinary Portland limestone cement.

In an embodiment, the tip of the third fibre is coated with a porous material, preferably, a carbonated cement layer.

In an embodiment, the tip of the fourth fibre is embedded in or coated with a porous material, preferably a cement layer.

In an embodiment, the tip of the fourth fibre is embedded in a range of 1mm to 1cm of cement paste. In an embodiment, the tip of the fourth fibre is coated with a thickness of 100 to 200 µm of cement paste, preferably 150 µm.

In an embodiment, the tip of the third fibre is coated in a porous carbonated cement layer, preferably the coating has a thickness of 5 to 200 µm, more preferably 150 µm.

In an embodiment, the tip of the third fibre is coated in a porous material, preferably the coating has a thickness higher than 1 µm.

In an embodiment, the tip of the fourth fibre is coated in a porous material, preferably a cement layer.

In an embodiment, the first, second, and third fibres are configured to operate with single-wavelength interrogation.

In an embodiment, the fourth fibre is configured to be interrogated using at least one light source at different wavelengths.

In an embodiment, the thickness of the iron film is 10 to 200 nm, preferably 50 to 150 nm, more preferably from 80 to 120 nm.

In an embodiment, the iron-based thin film is made of iron oxide or combinations thereof.

In an embodiment, the bundle is coated with a resin, preferably an epoxy resin.

In an embodiment, the number of light sources is at least 3.

In an embodiment, the light sources are light-emitting diodes (LED).

In an embodiment, the light sources have an intensity ranging from 200 to 1550 nm, preferably 350 to 1000 nm, more preferably 545, 650, and 750 nm.

In an embodiment, the cement layer, carbonated or non-carbonated, is 35%-70% water-to-cement weight ratio, preferably 45%-55% water-to-cement weight ratio.

In an embodiment, the cement layer covers:
5-100% of the area of the fibre tip; and
5-100% of the 10 µm directly above the fibre tip.

In an embodiment, the optical sensor further comprises:
a plate with holes or openings for receiving the tips of each fibre, to be inserted on the reinforced concrete structure;
a rod for receiving the fibre bundle;
at least one support for maintaining the alignment of each fibre of the fibre bundle and the optical component.

In an embodiment, the plate further comprises a polymeric mesh for distancing the cement layer of the third fibre when the sensor is in contact with a freshly concrete of the reinforced concrete structures.

In an embodiment, the rod is a steel rod.

In an embodiment, the plate is a polymeric plate.

In an embodiment, the support has a cross-section in form of Y, preferably the photodetector is placed in an edge of the Y and the light source is placed on the other edge of the Y.

It is also disclosed a method to obtain the optical sensor disclosed, comprising the following steps:
calibrating the first fibre tip, by embedding the fibre in air and in water and
monitoring the change in reflected light intensity;
coating or embedding the second fibre tip with non-carbonated cement layer calibrating the second fibre tip, by embedding it in air and in water;
coating third fibre tip with a carbonated cement layer,
calibrating the third fibre tip by varying the relative humidity in its surroundings;
coating the fourth fibre tip with an iron thin film and coating or embedding said tip in cement layer;
ensuring the fourth tip maintains the reflection when embedded in air and in water;
embedding the tip of the first fibre in the cure of the reinforced concrete structure;
embedding the second fibre in the reinforced concrete structure;
embedding the third fibre in the reinforced concrete structure;
embedding the fourth fibre in a reinforced concrete structure.

In an embodiment, the step of coating the fourth fibre tip with an iron thin film and coating or embedding said tip in cement layer is followed by verifying reflected light intensity.

In an embodiment, the iron thin film is an iron-based thin film.

It is also disclosed a method of monitoring the structural health of a reinforced concrete structure using the optical sensor disclosed above, comprising the steps of:
functionalizing the optical fibres according to claim 1;
embedding the optical fibre tips into fresh or cured concrete;
interrogating the fibres with light from a light source;
collecting reflected light from the fibres with the photodetector;comparing or monitoring the reflected intensity of the reflected light to determine curing state, humidity, water ingress, carbonation, and/or corrosion.

A "non-carbonated cement layer" refers to a cementitious coating applied to the fibre tip which has not undergone carbonation. In the context of the present invention, this denotes a layer composed of hydrated cement paste whose calcium-bearing phases (such as portlandite and calcium-silicate-hydrate) remain substantially unreacted with atmospheric carbon dioxide. The non-carbonated state provides a high alkalinity environment, enabling measurable changes in optical reflectance as carbonation progresses.

A "carbonated cement layer" refers to a cementitious coating that has been intentionally exposed to carbon dioxide so that its calcium-bearing phases are at least partially converted into calcium carbonate. The carbonation process changes porosity and modifies the refractive index and moisture absorption behaviour of the layer. In the present invention, such a carbonated cement layer is used as a humidity-responsive porous coating for relative-humidity sensing.

The term "porous material" relates to a material comprising interconnected pores of sufficient size to permit diffusion of water vapour or liquid water into the layer. In the present invention, the porous material is preferably a cement-based layer, such as carbonated or non-carbonated cement paste, but may alternatively include other porous inorganic or polymeric materials exhibiting comparable permeability and optical characteristics. The porosity enables variations in humidity or moisture content to alter the optical reflectance at the fibre tip.

Precast or fresh cement normally refers to cement that has not yet fully cured. Calibrating a sensor normally refers to determining or adjusting parameters by a user or a computer data processor where these parameters are used to adjust the values the sensor outputs to accurately reflect the real physical quantity being measured.

The requirement that the fibre tip coated with an iron-based thin film exhibits a measurable and stable reflected optical signal when interrogated with light is what can be designated as maintaining reflection. In the context of the manufacturing method, this means verifying that the reflectance amplitude at the iron-based coating remains detectable both in air and in water, ensuring that the corrosion-sensing mechanism is functional after coating and assembly.

### DETAILED DESCRIPTION

The present disclosure relates to an optical fibre sensor system for monitoring structural health in reinforced concrete structures. The system includes a bundle of multimode optical fibres, each functionalized to detect different degradation parameters such as curing, water intrusion, humidity, carbonation, and rebar corrosion. The fibres are interrogated using low-cost LEDs in the visible spectrum and integrated with photodetectors and 3D-printed holders for field durability. A multi-wavelength detection approach enables discrimination between corrosion and mechanical noise. The disclosure enables scalable, real-time, in-situ monitoring of concrete infrastructure using an embedded and cost-efficient optical sensing platform.

An optical fibre sensor for monitoring the integrity of reinforced concrete structures is disclosed. The sensor comprises a bundle of multimode optical fibres adapted for embedding in concrete, including a first bare fibre for refractive-index-based detection of curing and water ingress; a second fibre with a tip coated with a non-carbonated cement layer for detecting carbonation; a third fibre with a porous carbonated cement coating for sensing relative humidity; and a fourth fibre coated with an iron-based thin film and a cement layer for multi-wavelength corrosion detection. The solution further includes at least one light source coupled to each fibre, photodetectors for receiving reflected light, and a processing unit configured to correlate reflected intensity with structural integrity indicators.

In an embodiment, four sensors monitor changes in reflection at the fibre tip of a 600 µm multimode fibre (MMF) using LEDs and photodiodes, connected via a fibre bundle containing two 200 µm MMF.

Variations in the refractive index around the fibre tip are used to monitor water throughout the concrete lifecycle, including curing, RH changes and water intrusion.

Colorimetric changes in a cement paste layer and an iron-based thin film are used to monitor carbonation and corrosion.

The curing sensor is preferably temperature-independent and correlates strongly with cumulative heat release from cement hydration (R=0.95).

The RH sensor preferably monitors up to and beyond 100% RH, detecting water intrusion.

The corrosion sensor is able to detect early corrosion stages and distinguishes between reflection losses from corrosion and mechanical changes.

In an embodiment, the layer of cement paste for carbonation monitoring increases reflected intensity by 17% due to carbonation, with 63% of the increase occurring in 80 minutes in a 3% CO₂ atmosphere.

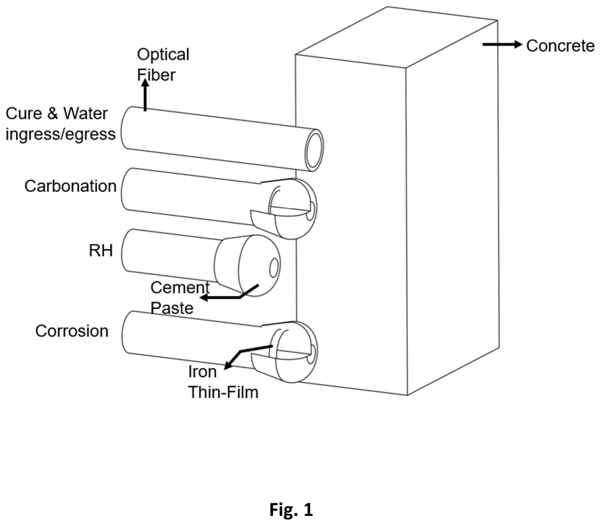

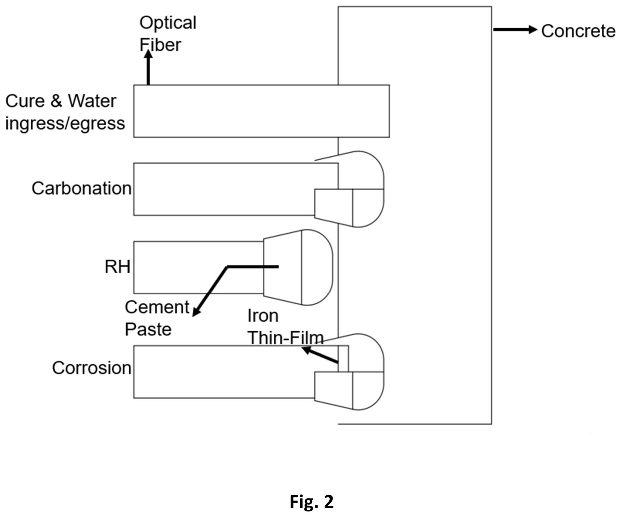

In an embodiment, Figures 1 and 2 show the sensing fibres embedded in concrete, highlighting the different degrees of interaction between each of the fibres and the structure.

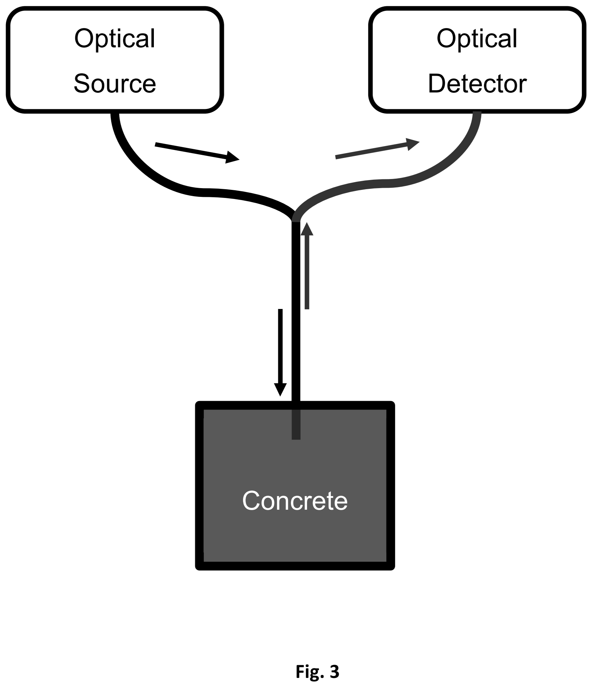

In an embodiment, Figure 3 shows a sensing scheme diagram where light from an optical source travel through a bundle of fibres interacts with the structure, is reflected back into an optical detector that measures it.

In an embodiment, four MMF of 600 µm diameter (FT600UMT, Thorlabs, Dortmund, Germany) are functionalized to monitor concrete curing, water intrusion, RH, carbonation, and rebar corrosion [3-6]. Each of the four fibres is interrogated by a fibre bundle consisting of two 200 µm multimode fibres (FT200UMT. Thorlabs, Dortmund, Germany) that carry light from the LED (B56L5111P, Roithner Lasertechnik, Wien, Austria) and into the photodetector (SPD400-0F, Roithner Lasertechnik, Wien, Austria).

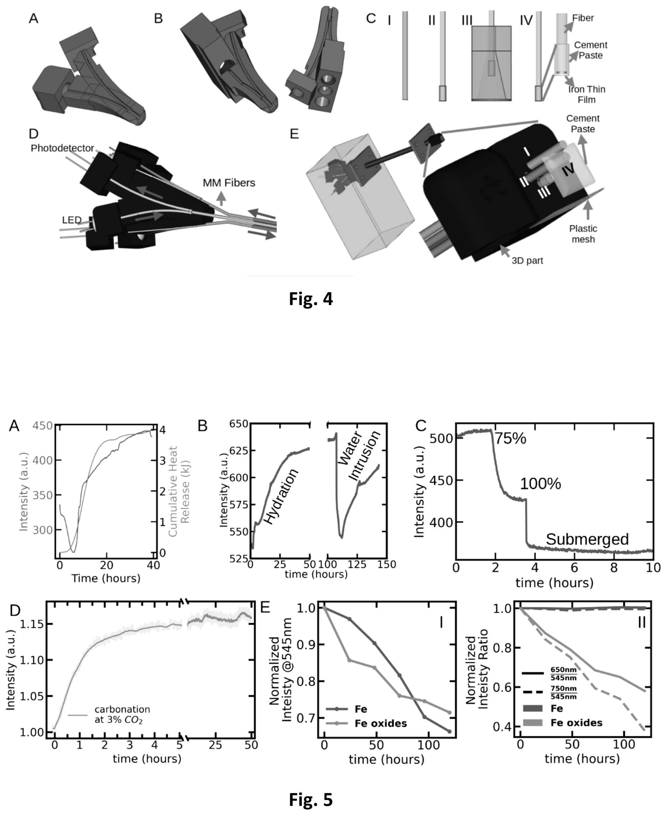

In an embodiment, Figure 4 represents: A) 3D printed part that holds the optical components for interrogation at a single wavelength and B) for several wavelengths using three different LEDs. C) Cross-section of each of the four multimode fibres monitors the structural health of reinforced concrete structures. D) 3D components used to interrogate the sensing fibres, and E) the complete sensor, with a container protecting the electronics, a steel rod to protect the sensing fibres, and the 3D part that joins all fibres of C).

All the optical components are preferably held in place using a 3D-printed part, Fig. 4A. A similar 3D-printed part is produced, in which three LEDs (LED545-04, ELD-650-523, LED750-03AU, Roithner Lasertechnik, Wien, Austria) are used to interrogate the sensing fibre, Fig. 4B.

A cross-section of each sensing fibre is presented in the embodiment of Fig. 4C, which shows from left to right: I - a bare polished fibre, used to monitor refractive index (RI) changes of water in the cement matrix of concrete; II - polished fibre covered in cement paste, embedded in a concrete structure to monitor carbonation by detecting increased reflection caused by the formation of CaCO₃; III - a polished fibre coated with cement paste, previously calibrated at different values of RH, each corresponding to an RI value from adsorbed water. A 3D-printed part and a plastic wire mesh are used to protect the calibrated cement paste matrix from changes caused by contact with the bulk cement paste of the concrete structure.; IV - a polished fibre tip coated with a 100 nm iron thin film, which changes reflectance in the visible spectrum due to iron oxide formation. Fibres I through III are interrogated using the setup shown in Fig. 4A, while fibre IV is interrogated with the setup shown in Fig. 4B. The need for interrogation at different wavelengths arises from the variability in monitoring iron oxidation using an iron thin film [6]. The four 3D parts are bundled together (Fig. 4D) and placed in a container (Fig. 4E). The four fibres pass through a steel rood, and the four tips displayed in Fig. 4C are placed inside a 3D-printed part at the end of the steel rood, as shown in the zoomed image in Fig. 4E.

Fig. 5 shows: A) Changes in reflected light intensity during concrete curing and the cumulative heat released. B) Decrease in reflected light intensity from water intrusion, which decreases the RI of the fibre tip vicinity. The effect of RH, C), and cement carbonation, D) on the reflected light intensity. E) Normalized reflected light intensity at a single wavelength, I, and ratio between the normalized intensity of several wavelengths, II.

The bare fibre tip is preferably used to monitor changes in the RI of the cement paste of reinforced concrete adjacent to it. During the curing process, an increase in reflected signal (indicating a decrease in RI) can be observed due to the consumption of water during cement hydration (Fig. 5A). Cement paste hydration is an exothermic reaction, and the cumulative heat release is strongly correlated with the change in the reflected signal (Fig. 5A, R=0.95).

This fibre also preferably monitors water intrusion (Fig. 5B). Water intrusion occupies the pores formed during hydration, leading to a signal decrease to values close to those observed at the start of hydration. A fibre tip functionalized with carbonated cement paste monitors changes in RH in the structure (Fig. 5C), with stable RH values achieved using saturated salt solutions. This information is also detectable in the bare fibre embedded in concrete; however, it is impossible to calibrate this tip by referencing stable RH values to reflectance intensity values from adsorbed water. Cement paste is used to functionalize a polished tip for monitoring cement paste carbonation. The reflected signal increases as the cement past changes to a lighter gray tone due to the formation of CaCO₃. In an atmosphere of 3% CO₂, the reflected signal increases by 17%, with 63% occurring in 80 minutes, Fig. 5D. This sensor is also sensitive to changes in RH and water intrusion. To compensate for this variability, the RH tip, which has been previously carbonated, will be used. The normalized intensity ratio of three LEDs (545, 650, and 750 nm) is used to detect corrosion of the thin iron film (Fig. 5E), induced by 3.5% salt water. As the iron film corrodes, less light from the LEDs is reflected at the tip. The similarity between signal decreases caused by corrosion and mechanical changes of the thin film is shown in Fig. 5E-I. Using different wavelengths and their intensity ratios allows differentiation between corrosion and variability associated with monitoring the reflection of a thin iron film (Fig. 5E-II).

Reinforced concrete structures are a cornerstone of society and human development, and monitoring their decay is essential. The present disclosure includes a low-cost sensor that monitors water in concrete by employing changes in reflection intensity caused by varying RI at the multimode fibre tip during curing, changes in RH and water intrusion. Water consumption during curing, monitored through changes in reflected light intensity and cumulative heat exchange from hydration, shows a strong correlation (R=0.95). A thin patch of cement (for example a thickness of 150 µm) is used as an indicator for concrete carbonation, changing color to a lighter gray tone. In a 3% CO₂, atmosphere, reflection increased by 17%, with 63% of the increase occurring within the first 80 minutes. The ratio of normalized reflected intensities of three LEDs demonstrated resilient against the variability of monitoring Fe corrosion, successfully distinguished between Fe corrosion and mechanical changes of the thin film.

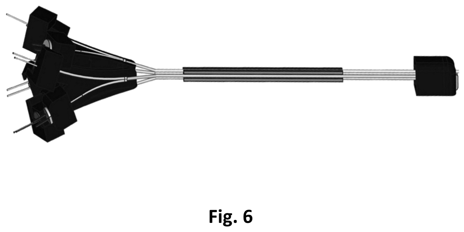

In an embodiment, Figure 6 shows a scheme of the optical fibre sensor for the structural health monitoring of reinforced concrete. Four optical fibres are used for the monitoring of reinforced concrete cure, water ingress/egress, carbonation, RH and rebar corrosion. Four independent fibre bundles connected to four LEDs and photodetectors are used to interrogate the system. A 3D part, in the middle, is used to align and secure the four fibres. Another 3D part, in the right, is used to guarantee the fibres interacts has expected with concrete.

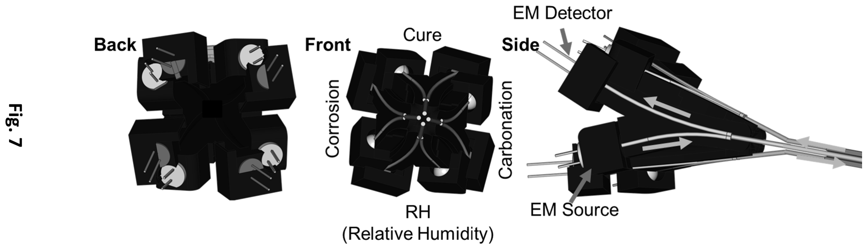

In an embodiment, Figure 7 shows scheme of the optical fibre sensor for the structural health monitoring of reinforced concrete. 3D printed ABS parts are used to align the optical components: electromagnetic (EM) waves source, EM waves detector and optical fibres.

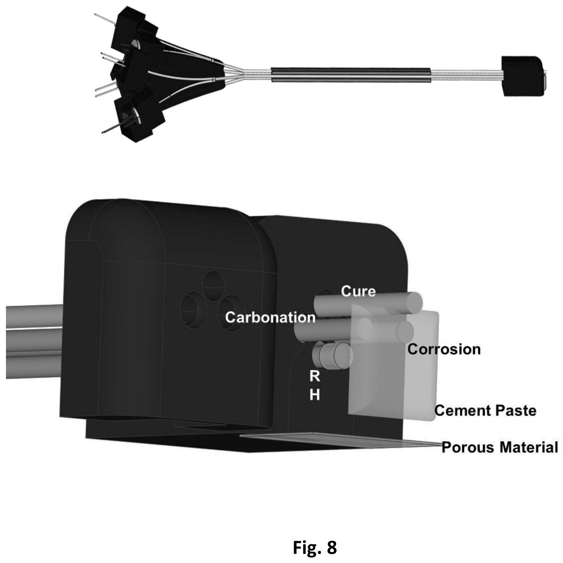

In an embodiment, Figure 8 represents a 3D printed ABS part used to position the sensing fibres inside of reinforced concrete. Fibre tip for cure monitoring is embedded directly into concrete. Carbonation tip is embedded in cement paste before being embedded in concrete. Corrosion tip has a thin-film of Fe and is embedded in cement paste before being embedded in concrete. RH tip is coated with cement paste and interacts with concrete at a distance through a porous material.

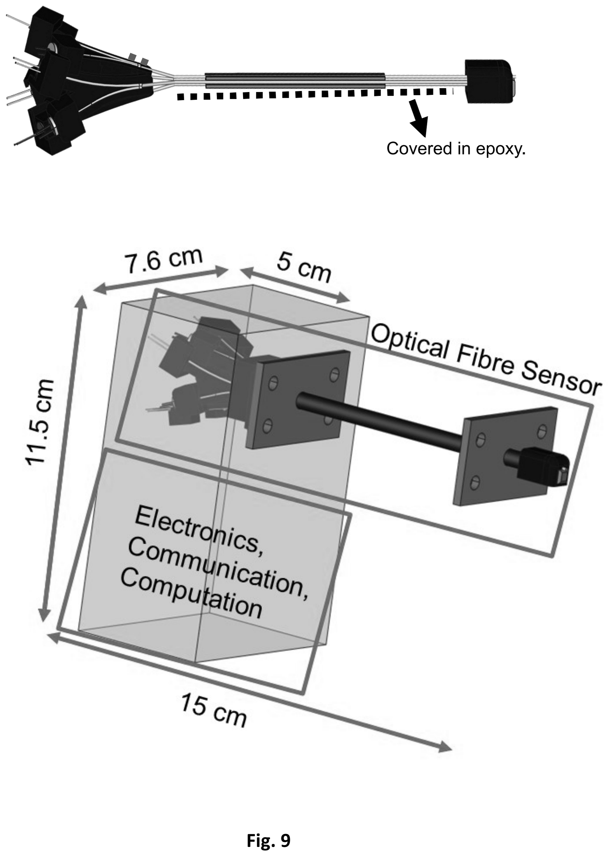

In an embodiment, Figura 9 represents an embodiment of an optical fibre sensor embedded in a steel piece to ensure its protections. Mechanical changes to the optical path of light are prevented by embedding the fibres in epoxy (dotted lines of the figure). Preferably, the epoxy is applied to the interior of the rod that houses the fibre bundles. This application ensures a uniform seal, eliminating any potential gaps that might permit fibre displacement or water infiltration, thereby protecting the embedded optoelectronic components.

In an embodiment, a plastic case is used to protect the electronics.

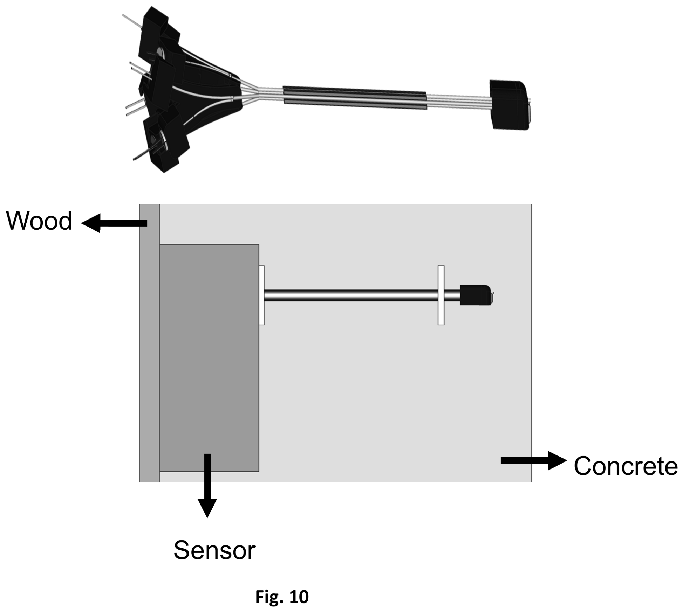

In an embodiment, Figure 10 represent sensors placed next to the external face of the reinforced concrete structure, allowing for the necessary repairs and changes that might be necessary during the sensors operating life. In an embodiment, the sensor is placed next to the external face of the reinforced concrete structure, allowing for the necessary repairs and changes that might be necessary during the sensors operating life.

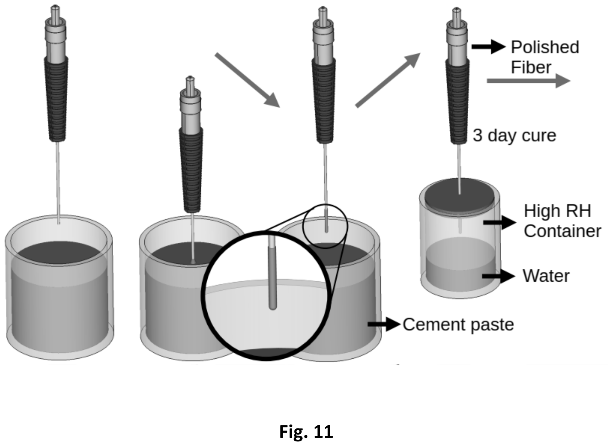

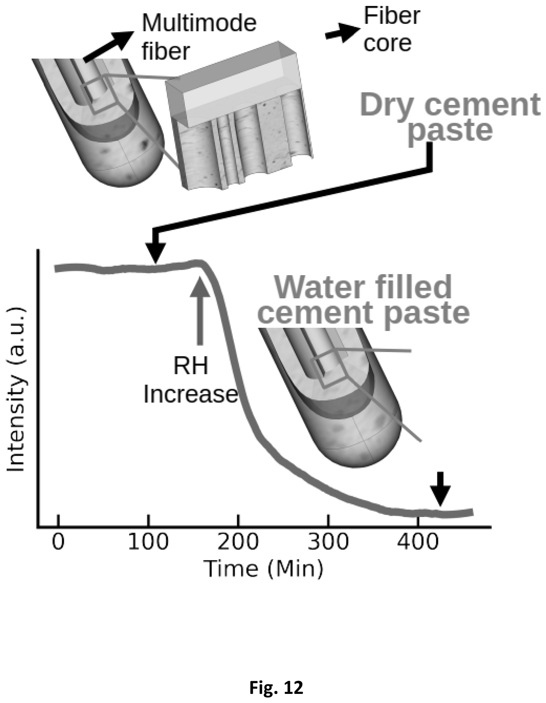

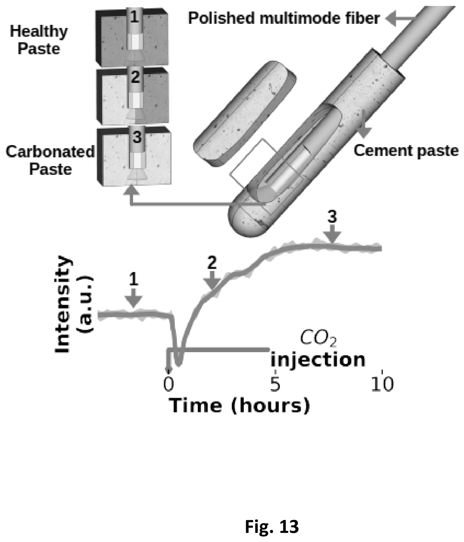

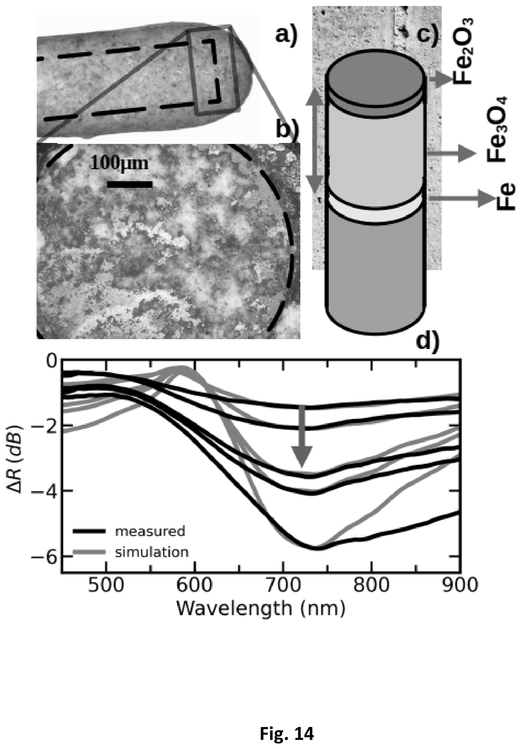

In an embodiment, Figure 11 represents the manufacturing of the RH sensors. A polished multimode fibre is embedded in freshly mixed cement paste and removed, ensuring it is coated with a thin layer of cement paste. Afterwords, it is placed in a 100% RH container to guarantee it is properly cured because for cement paste as thin as 150 µm, curing in open air is not a possibility, has the water will quickly evaporate, leaving the cure unfinished.

In an example 1, in the first 3 days of concrete curing, it is at 100% RH. However, the fibre that monitors 'water ingress and curing' can see water consumption in real time during the first 24 hours. After 48 hours, the curing fibre remains stable, but the RH fibre continues to measure the decrease in RH throughout the remaining curing process, which lasts 28 days or more. Both are complementary.

In an example 2, changes in the corrosion fibre with stable carbonation fibre means that there may have been chlorine intrusion. This is an important result for structures that are close to the sea or on roads in areas where it snows.

The present disclosure is co-financed by Component 5 - Capitalization and Business Innovation, integrated in the Resilience Dimension of the Recovery and Resilience Plan within the scope of the Recovery and Resilience Mechanism (MRR) of the European Union (EU), framed in the Next Generation EU, for the period 2021 - 2026, within project ATE, with reference 56 and by National Funds through the Portuguese funding agency, FCT - Fundação para a Ciência e a Tecnologia, within the PhD grant UI/BD/152695/2022 and the research contract CEECIND/00471/2017.

The term "comprising" whenever used in this document is intended to indicate the presence of stated features, integers, steps, components, but not to preclude the presence or addition of one or more other features, integers, steps, components or groups thereof.

Notably, the figures and examples above are not meant to limit the scope of the present disclosure to a single implementation, as other implementations are possible by way of interchange of some or all the described or illustrated elements. Moreover, where certain elements of the present disclosure can be partially or fully implemented using known components, only those portions of such known components that are necessary for an understanding of the present disclosure are described, and detailed descriptions of other portions of such known components are omitted so as not to obscure the disclosure. In the present specification, an implementation showing a singular component should not necessarily be limited to other implementations including a plurality of the same component, and vice-versa, unless explicitly stated otherwise herein. Moreover, applicants do not intend for any term in the specification or claims to be ascribed an uncommon or special meaning unless explicitly set forth as such. Further, the present disclosure encompasses present and future known equivalents to the known components referred to herein by way of illustration.

The foregoing description of the specific implementations will so fully reveal the general nature of the disclosure that others can, by applying knowledge within the skill of the relevant art(s), readily modify and/or adapt for various applications such specific implementations, without undue experimentation, without departing from the general concept of the present disclosure. Such adaptations and modifications are therefore intended to be within the meaning and range of equivalents of the disclosed implementations, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one skilled in the relevant art(s). It is to be understood that dimensions discussed or shown are drawings accordingly to one example and other dimensions can be used without departing from the disclosure.

The disclosure should not be seen in any way restricted to the embodiments described and a person with ordinary skill in the art will foresee many possibilities to modifications thereof.

The above described embodiments are combinable.

The following claims further set out particular embodiments of the disclosure.

### References

Aitcin, P.-C., "Cements of yesterday and today," Cement and Concrete Research 30(9), 1349-1359 (2000). 10.1016/S0008-8846(00)00365-3
Baroghel-Bouny, V., "Water vapour sorption experiments on hardened cementitious materials," Cement and Concrete Research 37(3), 414-437 (2007). 10.1016/j.cemconres.2006.11.019
da Silva, P. M., Coelho, L. C. C. and Almeida, J. M. M. M. de., "Single Fiber Reflectance Spectroscopy for the Monitoring of Cement Paste," Chemosensors 9(11), 312 (2021).10.3390/chemosensors9110312
da Silva, P. M., Coelho, L. C. C. and de Almeida, J. M. M. M., "Measuring Water Vapor Sorption Hysteresis of Cement Paste through an Optical Fiber Sensor," Chemosensors 11(2), 123 (2023). 10.3390/chemosensors11020123
da Silva, P. M., Mendes, J. P., Coelho, L. C. C. and de Almeida, J. M. M. M., "Real-Time Monitoring of Cement Paste Carbonation with In Situ Optical Fiber Sensors," Chemosensors 11(8), 449 (2023) .10.3390/chemosensors11080449
Da Silva, P. M., Carvalho, J. P. M., Mendes, J. P., De Almeida, J. M. M. M. and Coelho, L. C. C., "Monitoring Reinforced Concrete Structures Using Iron Thin Film Coated Optical fiber Sensors," EPJ Web Conf. 305, M. F. M. Costa and C. A. Ferreira Marques, Eds., 00024 (2024). 10.1051/epjconf/202430500024

## Claims

1. An optical fibre sensor for monitoring the integrity of a reinforced concrete structure, comprising a bundle of multimode optical fibres adapted to be embedded in the concrete structure, wherein said bundle comprises:
a first bare optical fibre for detecting changes in refractive index indicative of concrete curing and water ingress, configured for measuring reflectance;
a second optical fibre for detecting carbonation, comprising a tip coated with or embedded in a non-carbonated cement layer, configured for measuring reflectance;
a third optical fibre for detecting relative humidity changes, comprising a tip coated with a porous material, configured for measuring reflectance; and
a fourth optical fibre for detecting corrosion comprising a tip coated with an iron-based thin film, wherein said tip is further coated with or embedded in a porous material, wherein said fourth optical fibre is configured for measuring multi-wavelength reflectance at said iron-based thin film.

2. The optical sensor according to the previous claim, wherein said optical fibre sensor further comprises:
at least one light source optically coupled to each optical fibre of the fibre bundle;
at least one photodetector optically coupled to each optical fibre of the fibre bundle and configured to receive reflected light; and
a processing unit configured to analyse reflected light and to correlate the detected light with structural integrity indicators such as curing state, humidity, carbonation and corrosion.

3. The optical sensor according to any of the previous claims, wherein the tip of the third fibre is coated in a porous carbonated cement layer, preferably the coating has a thickness from 5 to 200 µm, more preferably about 150 µm; preferably the coating has a thickness higher than 1 µm.

4. The optical sensor according to any of the previous claims, wherein the tip of the fourth fibre is coated with or embedded in a cement layer.

5. The optical sensor according to any of the previous claims, wherein the sensor is configured to operate the first, second, and third fibres with single-wavelength illumination.

6. The optical sensor according to any of the previous claims, wherein the sensor is configured to operate the fourth fibre using at least one light source emitting at two or more wavelengths.

7. The optical sensor according to any of the previous claims, wherein the thickness of the iron-based film is 10 to 200 nm, preferably 50 to 150 nm, more preferably from 80 to 120 nm.

8. The optical sensor according to any of the previous claims, wherein the iron-based thin film is made of iron oxide and/or wherein the bundle is coated with a resin, preferably an epoxy resin.

9. The optical sensor according to claim 2 or claim 2 and any of the claims 3-8 wherein the light sources are light-emitting diodes (LED).

10. The optical sensor according to any of the previous claims, wherein the carbonated cement layer and/or the non-carbonated cement layer have a precast 35-70% water-to-cement weight ratio, preferably a precast 45-55% water-to-cement weight ratio.

11. The optical sensor according to any of the previous claims, wherein the cement layer covers 5-100% of the area of a corresponding fibre tip.

12. The optical sensor according to any of the previous claims, further comprising:
a plate with openings for receiving the tips of each said fibre, to be inserted on the reinforced concrete structure;
a rod for receiving the fibre bundle;
at least one support for maintaining the alignment of each said fibre of the fibre bundle and an optical component.

13. The optical sensor according to the previous claim, wherein the plate further comprises a polymeric mesh for distancing the cement layer of the third optical fibre when the sensor is in contact with precast concrete of the reinforced concrete structures.

14. A method to obtain the optical sensor according to any of the previous claims comprising the following steps:
calibrating the first optical fibre tip, by arranging the first optical fibre in air and, subsequently or previously, in water and monitoring the change in reflected light intensity to calibrate the first optical fibre;
coating or embedding the second fibre tip with non-carbonated cement layer and calibrating the second optical fibre tip by embedding it in air and, subsequently or previously, in water and monitoring the change in reflected light intensity to calibrate the second optical fibre;
coating the third optical fibre tip with a carbonated cement layer and calibrating the third optical fibre tip by varying the relative humidity in its surroundings and monitoring the change in reflected light intensity to calibrate the third optical fibre; coating the fourth optical fibre tip with an iron-based thin film and coating or embedding said tip in a cement layer, followed by verifying reflected light intensity.

15. A method of monitoring the structural health of a reinforced concrete structure using the optical sensor according to any of the previous claims 1 to 13 comprising the steps of:
embedding the tips of the optical fibre bundle into fresh or cured concrete;
interrogating the optical fibres with light from one or more light sources;
collecting reflected light from the optical fibres with a photodetector;
monitoring reflected intensity of the reflected light to determine curing state, humidity, water ingress, carbonation, and/or corrosion.
